# EUROPEAN PATENT SPECIFICATION

(11) **EP 3 100 733 B1**
(45) Date of publication and mention of the grant of the patent: **12.12.2018**
(21) Application number: 15743600.7
(22) Date of filing: 28.01.2015
(51) Int. Cl.: A61K 31/551, A61K 9/70, A61K 47/18, A61K 47/32, A61K 47/34, A61P 37/08

(54) **EMEDASTINE-CONTAINING TAPE**
EMEDASTINHALTIGES BAND
RUBAN ADHÉSIF CONTENANT DE L'EMÉDASTINE

(30) Priority: 31.01.2014 JP 2014016983
(43) Date of publication of application: 07.12.2016
(73) Proprietor: Hisamitsu Pharmaceutical Co., Inc., Tosu-shi, Saga 841-0017 (JP)
(72) Inventor: MICHINAKA, Yasunari, Tsukuba-shi Ibaraki 305-0856 (JP); SHINODA, Tomohiro, Tsukuba-shi Ibaraki 305-0856 (JP); IMAMURA, Kana, Tsukuba-shi Ibaraki 305-0856 (JP)
(74) Representative: Grünecker Patent- und Rechtsanwälte PartG mbB
(86) International application number: PCT/JP2015/052393
(87) International publication number: WO 2015/115497

(56) References cited:
- EP-A1- 1 270 205
- EP-A1- 1 872 784
- WO-A1-2005/115355
- WO-A1-2012/144405
- JP-A- 2008 127 351
- JP-A- 2008 156 346
- BIELORY LEONARD ET AL: "Efficacy and tolerability of newer antihistamines in the treatment of allergic conjunctivitis", D, ADIS INTERNATIONAL LTD, NZ, vol. 65, no. 2, 1 January 2005 (2005-01-01), pages 215-228, XP009114120, ISSN: 0012-6667, DOI: 10.2165/00003495-200565020-00004

## Description

### Technical Field

The present invention relates to an emedastine-containing tape preparation.

### Background Art

Emedastine fumarate has potent and selective antihistamine action, and is also recognized to have evident antiallergic action, action of suppressing increase in nasal respiratory resistance, and action of suppressing eosinophilic infiltration into the nasal mucosa in experimental allergic models. Emedastine fumarate also has in vitro action of suppressing substance P-stimulated release of histamine from mast cells, and in vitro action of inhibiting migration of eosinophils due to platelet-activating factor and leukotriene B₄.

As oral preparations of emedastine fumarate, DAREN (registered trademark, Merck Sharp & Dohme Besloten Vennootschap) capsules, REMICUT (registered trademark, Merck Sharp & Dohme Besloten Vennootschap) capsules, and the like are commercially available (for example, Patent Literature 1 and Non Patent Literature 1).

The oral preparations of emedastine fumarate show a wide range of variances in blood concentration, and thus, problems of the preparations is that they may express side effects such as drowsiness, and may not provide sufficient pharmacological effects. In recent years, therefore, the development of various preparations containing emedastine has been attempted (Patent Literatures 2 to 5). Patent Literature 2 discloses a composition for percutaneous administration comprising emedastine and an lower alkyl aliphatic monocarboxylate or an di-lower alkyl aliphatic dicarboxylate, and specifically describes oily ointments, gels, creams, lotions, and sprays as examples thereof. Patent Literature 3 discloses an adhesive patch wherein an adhesive layer made of an acrylic-based adhesive base, a silicone-based adhesive base, or a rubber-based adhesive base and emedastine is laminated on one surface of a support layer. Patent Literature 4 discloses an emedastine patch preparation having a pressure-sensitive adhesive layer containing emedastine and an acrylic polymer substantially free of acidic functional groups.

### Citation List

### Patent Literature

Patent Literature 1: JP S58-79983 A
Patent Literature 2: JP H3-83924 A
Patent Literature 3: JP H7-33665 A
Patent Literature 4: JP H8-193030 A
Patent Literature 5: JP 2007-186500 A
Patent Literature 6: JP 2008-127351 A

### Non Patent Literature

Non Patent Literature 1: DAREN Capsule Pharmaceutical Interview Form

### Summary of Invention

### Technical Problem

The present inventors have made the following finding concerning patches containing emedastine: a patch that uses a pressure-sensitive adhesive layer comprising an acrylate pressure-sensitive adhesive exhibits excellent skin penetrability of emedastine, but strong adhesion of the pressure-sensitive adhesive layer may cause a problem at the time of adhesion or peeling; on the other hand, a patch that uses a pressure-sensitive adhesive layer comprising a rubber-based pressure-sensitive adhesive is inferior in the skin penetrability of emedastine, and may not have sufficient anchoring properties on the support film of the pressure-sensitive adhesive layer.

On the other hand, Patent Literature 5 discloses a patch preparation wherein a pressure-sensitive adhesive layer comprising a branched monoalcohol with 12 to 28 carbon atoms, a drug that is liquid at or around room temperature (excluding, however, the free base of bisoprolol), and a polyisobutylene-based pressure sensitive adhesive is laminated on one surface of a support. In particular, Patent Literature 5 discloses that the anchoring properties are improved through the use of a laminated film of a non-porous plastic film and a porous film as the support. Patent Literature 6 discloses a patch preparation comprising a support, and a pressure-sensitive adhesive layer comprising a drug and laminated on at least one surface of the support, wherein the support includes a polyester film with a thickness of 0.5 to 6.0 µm and a polyester nonwoven fabric directly bound to the film and also discloses that the patch preparation is improved in the anchoring properties.

The patches described in Patent Literatures 5 and 6, however, required the use of a support formed by laminating a plurality of films or nonwoven fabrics, in order to improve the anchoring properties. Thus, in view of the above-described circumstances, the present invention aims to provide an emedastine-containing tape preparation excellent in the skin penetrability of the drug and anchoring properties.

### Solution to Problem

As a result of extensive research, the present inventors have found that when a pressure-sensitive adhesive layer comprises emedastine, a fumarate salt of an organic amine, and a pressure-sensitive adhesive, excellent skin penetrability of emedastine and anchoring properties are provided, and thus have completed the present invention. The present invention therefore provides an emedastine-containing tape preparation comprising a support film, and a pressure-sensitive adhesive layer laminated on the support film, the pressure-sensitive adhesive layer comprising emedastine, a fumarate salt of an organic amine, and a pressure-sensitive adhesive.

From the viewpoint of further improvement in the skin penetrability of emedastine and anchoring properties, it is preferable that the above-described organic amine comprise an organic amine selected from the group consisting of monoethanolamine, diethanolamine, triethanolamine, monoisopropanolamine, diisopropanolamine, and polyethyleneimine, and it is more preferable that the organic amine comprises monoethanolamine or diethanolamine.

Moreover, from the viewpoint of further improvement in the anchoring properties, it is preferable that the material of the above-described support film comprise polyethylene terephthalate (PET) or polyethylene (PE). When the support film comprises polyethylene terephthalate or polyethylene, the anchoring properties are further improved.

From the viewpoint of further improvement in the skin penetrability of emedastine, it is preferable that the above-described pressure-sensitive adhesive comprises a rubber-based pressure-sensitive adhesive, and particularly a styrene-isoprene-styrene block copolymer (SIS).

### Advantageous Effects of Invention

The emedastine-containing tape preparation according to the present invention is excellent not only in the skin penetrability of emedastine and anchoring properties, but also in the dissolution properties of emedastine and peel strength. Furthermore, in the emedastine-containing tape preparation according to the present invention, the anchoring properties can be easily improved without an additional layer for improving the anchoring properties in the support film or pressure-sensitive adhesive layer.

### Brief Description of Drawings

Figure 1 is a graph showing the results of a dissolution test.
Figure 2 is a graph showing the results of a skin penetrability test.
Figure 3 is a graph showing the results of a skin penetrability test.
Figure 4 is a graph showing the results of a peel test.
Figure 5 is a graph showing the results of an anchoring property test 1.
Figure 6 is a graph showing the results of an anchoring property test 2.

### Description of Embodiments

An emedastine-containing tape preparation according to one embodiment of the present invention comprises a support film, and a pressure-sensitive adhesive layer laminated on the support film, the pressure-sensitive adhesive layer comprising emedastine, a fumarate salt of an organic amine selected from the group consisting of monoethanolamine, diethanolamine, triethanolamine, monoisopropanolamine, and polyethyleneimine, and a pressure-sensitive adhesive.

The material of the above-described support film is not particularly limited so long as it is suitable for supporting the pressure-sensitive adhesive layer, and an elastic or non-elastic material can be used. Polyethylene (PE), polypropylene, polybutadiene, ethylene vinyl acetate copolymer, polyvinyl chloride, polyester, nylon, polyurethane, polyacrylonitrile (PAN), or the like can be used in the form of a film, a sheet, a laminate thereof, a porous material, foams, a fabric and a nonwoven fabric, as well as a laminated product thereof. Moreover, it is preferable that the material of the above-described support film comprise polyethylene terephthalate (PET) or polyethylene. Specific examples of preferable support films include the Scotchpak (registered trademark, 3M Company) series such as Scotchpak 9723 and 9732.

The above-described support film may be subjected to a sand matting treatment on its surface that is to be contacted with the pressure-sensitive adhesive layer. The anchoring properties are further enhanced by subjecting the support film to a sand matting treatment The sand matting treatment can be performed using a method well known to those skilled in the art.

Emedastine is also referred to as 1-(2-ethoxyethyl)-2-(4-methyl-1,4-diazepan-1-yl)-1H-benzimidazole, and has a molecular weight of 302.41. The emedastine used in this embodiment may be emedastine in free form or a pharmaceutically acceptable salt thereof. Examples of pharmaceutically acceptable salts of emedastine include inorganic acid salts such as hydrochloride, hydrobromide, and phosphate; carboxylates such as acetate, propionate, fumarate, maleate, citrate, and glutamate; sulfonates such as methanesulfonate, benzenesulfonate, and toluenesulfonate; and carbonate. The acid dissociation constants (pKa) of emedastine are 4.51 and 8.48, and emedastine can be in a salt form in which one or two molecules of an acid per molecule of emedastine have been added. Emedastine may be in the form of an acid addition salt of one molecule or an acid addition salt of two molecules. The pharmaceutically acceptable salt of emedastine is, for example, a difumarate salt represented by the following chemical formula (1):

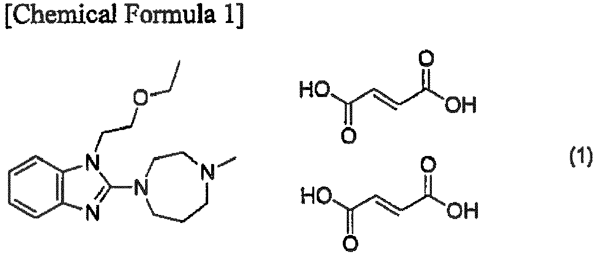

The content of emedastine is preferably 0.1 to 40 mass%, more preferably 0.1 to 20 mass%, and particularly preferably 1 to 10 mass%, based on the total mass of the pressure-sensitive adhesive layer.

The fumarate salt of an organic amine is selected from the group consisting of monoethanolamine (pKa: 9.5), diethanolamine (pKa: 8.9), triethanolamine (pKa: 7.5), monoisopropanolamine (pKa: 11.3), and polyethyleneimine (pKa: approximately 8.7). In particular, it is more preferable that the pressure-sensitive adhesive layer comprise a fumarate salt of monoethanolamine or a fumarate salt of diethanolamine, because the preparation is unlikely to be colored, and the skin penetrability of emedastine is further enhanced. Specific examples of polyethyleneimine include EPOMIN SP-003, SP-006, SP-012, SP-018, SP-200, and P-1000 (registered trademark, Nippon Shokubai Co., Ltd.). Specific examples of aminoalkyl methacrylate copolymer E include Eudragit E (registered trademark, Evonik Roehm GmbH). These organic amines may be used singly or in combinations of two or more thereof.

From the viewpoint of improving the anchoring properties, it is preferable that the fumarate salt of an organic amine be formed by mixing emedastine fumarate and the organic amine. That is, an organic amine can be used which causes salt exchange when the basicity of the organic amine is compared with that of emedastine.

When the amount of emedastine contained in the tape preparation is represented by the number of moles, the content of the organic amine is preferably a weight corresponding to 2 to 6 molar equivalents, and more preferably a weight corresponding to 3 to 5 molar equivalents, relative to the number of moles of emedastine. When the fumarate salt of an organic amine and emedastine are used, the preferable content of the organic amine described above may be set within a range that can be calculated based on the molecular weight.

As the pressure-sensitive adhesive, a rubber-based pressure-sensitive adhesive, an acrylate-based pressure-sensitive adhesive, or a silicone-based pressure-sensitive adhesive can be used.

Examples of rubber-based pressure-sensitive adhesives include styrene-isoprene-styrene block copolymer (SIS), isoprene rubber, polyisobutylene (PIB), styrene-butadiene-styrene block copolymer (SBS), styrene-butadiene rubber (SBR), and polysiloxane. These rubber-based pressure-sensitive adhesives may be used singly or in combinations of two or more thereof. Examples of preferable rubber-based pressure-sensitive adhesives include SIS and PIB. Specific examples of rubber-based pressure-sensitive adhesives include Oppanol B12, B15, B50, B80, B100, B120, B150, and B220 (trade names, manufactured by BASF SE), JSR BUTYL 065, 268, and 365 (trade names, manufactured by JSR Corporation), Vistanex LM-MS, MH, H, MML-80, 100, 120, and 140 (trade names, manufactured by Exxon Chemical Corporation), HYCAR (trade name, manufactured by Goodrich Corporation), and SIBSTAR T102 (trade name, manufactured by Kaneka Corporation).

When a rubber-based pressure-sensitive adhesive is used as the pressure-sensitive adhesive, the content of the rubber-based pressure-sensitive adhesive is preferably 5 to 99.9%, and more preferably 10 to 97%, based on the mass of the entire pressure-sensitive adhesive layer. In particular, when the rubber-based pressure-sensitive adhesive includes SIS, the content of the rubber-based pressure-sensitive adhesive is preferably 5 to 50%, and more preferably 10 to 40%, based on the mass of the entire pressure-sensitive adhesive layer. On the other hand, when the rubber-based pressure-sensitive adhesive does not include SIS, the content of the rubber-based pressure-sensitive adhesive is preferably 50 to 99.9%, and more preferably 70 to 97%, based on the mass of the entire pressure-sensitive adhesive layer.

The acrylate-based pressure-sensitive adhesive may be a polymer containing an acrylic ester as a monomer unit. The acrylate-based pressure-sensitive adhesive may be a homopolymer, or a copolymer obtained by copolymerization of two or more acrylic esters. Moreover, the acrylate-based pressure-sensitive adhesive may also contain, in addition to the acrylic ester, monomer units such as acrylic acid, acrylamide, vinyl acetate, vinyl alcohol, and styrene. Examples of acrylic esters include methyl acrylate and 2-ethylhexyl acrylate, and examples of acrylic amides include N,N-dimethyl acrylamide and N-acryloyl morpholine.

The acrylate-based pressure-sensitive adhesive is preferably an acrylate pressure-sensitive adhesive having hydroxy groups or an acrylate pressure-sensitive adhesive not having a polar functional group, and more preferably an acrylate pressure-sensitive adhesive having hydroxy groups. When the acrylate-based pressure-sensitive adhesive is an acrylate pressure-sensitive adhesive having hydroxy groups or an acrylate pressure-sensitive adhesive not having a polar functional group, the anchoring properties of the pressure-sensitive adhesive layer can be further improved in the cases where the material of the support film is any of polyethylene terephthalate (PET), polyethylene (PE), and ethylene vinyl acetate (EVA). In particular, when the acrylate-based pressure-sensitive adhesive is an acrylate pressure-sensitive adhesive having hydroxy groups, the effect of improving the anchoring properties is demonstrated more remarkably.

The acrylate pressure-sensitive adhesive having hydroxy groups means a polymer containing, as a monomer unit, an alkyl acrylate ester in which a carbon atom on the alkyl group thereof has hydroxy groups, and specific examples thereof include DURO-TAK (registered trademark) 387-2510, DURO-TAK (registered trademark) 87-2510, DURO-TAK (registered trademark) 387-2287, DURO-TAK (registered trademark) 87-2287, DURO-TAK (registered trademark) 87-4287, GELVA (registered trademark) GMS 788, DURO-TAK (registered trademark) 387-2516, DURO-TAK (registered trademark) 87-2516, and DURO-TAK (registered trademark) 87-2074.

The acrylate pressure-sensitive adhesive not having a polar functional group means a polymer containing, as a monomer unit, an acrylate ester in which a carbon atom on the alkyl group of thereof do not have a polar functional group. As used herein, the polar functional group is a group that acts as a hydrogen donor or hydrogen acceptor, such as hydroxy, carboxy, amino, and carbamoyl groups. Specific examples of acrylate pressure-sensitive adhesives not having a polar functional group include DURO-TAK (registered trademark) 87-900A, DURO-TAK (registered trademark) 87-901A, DURO-TAK (registered trademark) 87-9301, DURO-TAK (registered trademark) 87-4098, GELVA (registered trademark) GMS 3083, and GELVA (registered trademark) GMS 3253 (all manufactured by Henkel Corporation), MAS811 (CosMED Pharmaceutical Co., Ltd.), and MAS683 (CosMED Pharmaceutical Co., Ltd.).

When an acrylate-based pressure-sensitive adhesive is used as the pressure-sensitive adhesive, the content of the acrylate-based pressure-sensitive adhesive is preferably 50 to 99.9%, and more preferably 70 to 97%, based on the mass of the entire pressure-sensitive adhesive layer.

The silicone-based pressure-sensitive adhesive may be a silicone-based pressure-sensitive adhesive that is cured by irradiation with light or the like, or cured by moisture. Examples of silicone-based pressure-sensitive adhesives include MD7-4502 Silicone Adhesive, MD7-4602 Silicone Adhesive, BIO-PSA 7-4301 Silicone Adhesive, BIO-PSA 7-4302 Silicone Adhesive, BIO-PSA 7-4201 Silicone Adhesive, BIO-PSA 7-4202 Silicone Adhesive, BIO-PSA 7-4101 Silicone Adhesive, BIO-PSA 7-4102 Silicone Adhesive, BIO-PSA 7-4601 Silicone Adhesive, BIO-PSA 7-4602 Silicone Adhesive, BIO-PSA 7-4501 Silicone Adhesive, BIO-PSA 7-4502 Silicone Adhesive, BIO-PSA 7-4401 Silicone Adhesive, BIO-PSA 7-4402 Silicone Adhesive, 7-9800A, 7-9800B, 7-9700A, and 7-9700B (all manufactured by Dow Corning Corporation). Although a tape preparation comprising a silicone-based pressure-sensitive adhesive as the pressure-sensitive adhesive may experience anchoring failure particularly when the material of the support film is ethylene vinyl acetate (EVA), the remarkable effect of improving the anchoring properties is exhibited if the pressure-sensitive adhesive layer comprises the fumarate salt of an organic amine.

When a silicone-based pressure-sensitive adhesive is used as the pressure-sensitive adhesive, the content of the silicone-based pressure-sensitive adhesive is preferably 50 to 99.9%, and more preferably 70 to 97%, based on the mass of the entire pressure-sensitive adhesive layer.

The pressure-sensitive adhesive layer may also contain additives such as a tackifier resin, a plasticizer, a percutaneous absorption-promoting agent, a stabilizer, a filler, and a perfume.

Examples of the above-described tackifier resin include rosin, rosin derivatives such as glycerol ester of rosin, hydrogenated rosin, glycerol ester of hydrogenated rosin, and pentaerythritol ester of rosin; an alicyclic saturated hydrocarbon resin such as Arkon P100 (trade name, Arakawa Chemical Industries, Ltd.); an aliphatic hydrocarbon resin such as Quintone B170 (trade name, Zeon Corporation); a terpene resin such as Clearon P-125 (trade name, Yasuhara Chemical Co., Ltd.); and a maleic acid resin. Among these, particularly, glycerol ester of hydrogenated rosin, an alicyclic saturated hydrocarbon resin, an aliphatic hydrocarbon resin, and a terpene resin are preferable. These tackifier resins may be used singly or in combinations of two or more thereof. The inclusion of a tackifier resin enhances the adhesiveness of the pressure-sensitive adhesive layer, which allows various other physical properties to be stably maintained.

The content of the above-described tackifier resin is preferably 20 to 70 mass%, and more preferably 30 to 60 mass%, based on the total mass of the pressure-sensitive adhesive layer.

Examples of the above-described plasticizer include petroleum-based oils such as paraffinic process oils, naphthenic process oils, and aromatic process oils; squalane; squalene; plant-based oils such as olive oil, camellia oil, castor oil, tall oil, and peanut oil; silicone oils; dibasic acid esters such as dibutyl phthalate and dioctyl phthalate; liquid rubbers such as polybutene and liquid isoprene rubber; liquid fatty acid esters such as isopropyl myristate, hexyl laurate, diethyl sebacate, and diisopropyl sebacate; diethylene glycol; polyethylene glycol; glycol salicylate; propylene glycol; dipropylene glycol; triacetin; triethyl citrate; and crotamiton. Among these, liquid paraffins, liquid polybutene, isopropyl myristate, diethyl sebacate, and hexyl laurate are preferable as the plasticizer, and particularly, liquid polybutene, isopropyl myristate, and liquid paraffins are preferable. A mixture of two or more of these plasticizers may be used.

The content of the above-described plasticizer is preferably 5 to 40 mass%, and more preferably 10 to 30 mass%, based on the total mass of the pressure-sensitive adhesive layer.

As the above-described percutaneous absorption-promoting agent, any compound that is conventionally known to have percutaneous absorption-promoting action on the skin may be used. Examples of percutaneous absorption-promoting agents include organic acids, fatty acids having 6 to 20 carbon atoms, fatty alcohols, fatty acid esters, fatty acid amides and ethers, aromatic organic acids, aromatic alcohols, aromatic organic acid esters and ethers (these may be a saturated or unsaturated compound, and may be any of cyclic, linear, and branched compounds), lactic acid esters, acetic acid esters, monoterpene-based compounds, sesquiterpene-based compounds, Azone, Azone derivatives, pirotiodecane, glycerol fatty acid esters, propylene glycol fatty acid esters, sorbitan fatty acid esters (Span), polysorbates (Tween), polyethylene glycol fatty acid esters, polyoxyethylene hydrogenated castor oils (HCO), polyoxyethylene alkyl ethers, sucrose fatty acid esters, and plant oils. Specific examples of percutaneous absorption-promoting agents include lauric acid, myristic acid, palmitic acid, stearic acid, isostearic acid, oleic acid, linolic acid, linolenic acid, lauryl alcohol, myristyl alcohol, oleyl alcohol, isostearyl alcohol, cetyl alcohol, methyl laurate, hexyl laurate, lauric acid diethanolamide, isopropyl myristate, myristyl myristate, octyldodecyl myristate, cetyl palmitate, methyl salicylate, ethylene glycol salicylate, cinnamic acid, methyl cinnamate, cresol, cetyl lactate, lauryl lactate, ethyl acetate, propyl acetate, geraniol, thymol, eugenol, terpineol, 1-menthol, borneol, d-limonene, isoeugenol, isoborneol, nerol, dl-camphor, glycerol monocaprylate, glycerol monocaprate, glycerol monolaurate, glycerol monooleate, sorbitan monolaurate, sucrose monolaurate, polysorbate 20, propylene glycol, propylene glycol monolaurate, polyethylene glycol monolaurate, polyethylene glycol monostearate, polyoxyethylene lauryl ether, HCO-60, pirotiodecane, and olive oil.

The content of the above-described percutaneous absorption-promoting agent is preferably 0 to 30 mass%, and more preferably 0 to 15 mass%, based on the total mass of the pressure-sensitive adhesive layer.

Examples of the above-described stabilizer include antioxidants (tocopherol derivatives, ascorbic acid derivatives, erythorbic acid derivatives, nordihydroguaiaretic acid, gallic acid derivatives, dibutylhydroxytoluene (BHT), butylated hydroxyanisole, sodium pyrosulfite, sodium sulfite, and the like), and ultraviolet absorbents (imidazole derivatives, benzotriazol derivatives, p-aminobenzoic acid derivatives, anthranilic acid derivatives, salicylic acid derivatives, cinnamic acid derivatives, benzophenone derivatives, coumalic acid derivatives, camphor derivatives, and the like).

The content of the above-described stabilizer is preferably 0 to 20 mass%, and more preferably 0 to 10 mass%, based on the total mass of the pressure-sensitive adhesive layer.

Examples of the above-described filler include metal oxides (zinc oxide, titanium oxide, and the like), metal salts (calcium carbonate, magnesium carbonate, zinc stearate, and the like), silicate compounds (kaolin, talc, bentonite, Aerosil, hydrous silica, aluminum silicate, magnesium silicate, magnesium aluminometasilicate, and the like), and metal hydroxides (aluminum hydroxide and the like).

The content of the above-described filler is preferably 0 to 20 mass%, and more preferably 0 to 10 mass%, based on the total mass of the pressure-sensitive adhesive layer.

The pressure-sensitive adhesive layer may include a solvent. Examples of the above-described solvent include methanol, ethanol, acetone, ethyl acetate, toluene, tetrahydrofuran, and acetonitrile.

The tape preparation according to this embodiment may also comprise a releasing film for covering and protecting the pressure-sensitive adhesive layer.

As the material of the above-described releasing film, a film of a polyester (polyethylene terephthalate, polyethylene naphthalate, polybutylene terephthalate, or the like), a polyolefin (polyethylene, polypropylene, or the like), or paper or the like can be used, and a material that has been subjected to a mold release treatment by coating its surface that is abut on the pressure-sensitive adhesive layer with a silicone, Teflon (registered trademark), or the like is preferable, and particularly, a silicone-treated polyethylene terephthalate film is suitably used.

The thickness of the pressure-sensitive adhesive layer is preferably 30 to 300 µm, and more preferably 50 to 200 µm. Moreover, the area of the adhesive surface of the pressure-sensitive adhesive layer is preferably 1 to 100 cm², and more preferably 3 to 40 cm².

### (Method for producing the tape preparation)

The tape preparation according to this embodiment can be produced using a method known to those skilled in the art. For example, a pressure-sensitive adhesive, emedastine, a fumarate salt of the above mentioned organic amine, and a solvent are mixed, and optionally, additives such as a tackifier resin, a plasticizer, a percutaneous absorption-promoting agent, a stabilizer, a filler, and a perfume are further added thereto and mixed using a mixer to prepare a homogeneous coating solution. The obtained coating solution is spread onto a film (releasing film) that has been subjected to the mold release treatment; the solvent is removed by drying to form a pressure-sensitive adhesive layer; a support film is then laminated thereon, and the support film and the pressure-sensitive adhesive layer are pressed and bonded each other, to thereby obtain a tape preparation. In the tape preparation thus obtained, the releasing film, the pressure-sensitive adhesive layer, and the support are laminated in order.

### Examples

The present invention will be hereinafter described in detail with reference to examples and comparative examples.

### Production of tape preparations according to Examples 1 to 8 and Comparative Examples 1 to 3

In accordance with the proportions set forth in Table 1 or 2, a styrene-isoprene-styrene block copolymer (SIS), an alicyclic saturated hydrocarbon resin, a liquid paraffin, emedastine fumarate or emedastine, and a fumarate salt of an organic amine or sodium hydroxide were added to toluene, and then the resultant was mixed using a mixer to prepare a homogeneous coating solution. Note that the values shown in Tables 1 and 2 each correspond to the percentage by mass (unit: w/w%) of each of the components in the pressure-sensitive adhesive layer. The obtained coating solution was spread onto a film (releasing film) that had been subjected to the mold release treatment, the solvent was removed by drying to form a pressure-sensitive adhesive layer, a support film was then laminated thereon, and the support film and the pressure-sensitive adhesive layer were pressed and bonded to each other, to thereby obtain a tape preparation. In the obtained tape preparation, therefore, the releasing film, the pressure-sensitive adhesive layer, and the support were laminated in order.

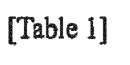

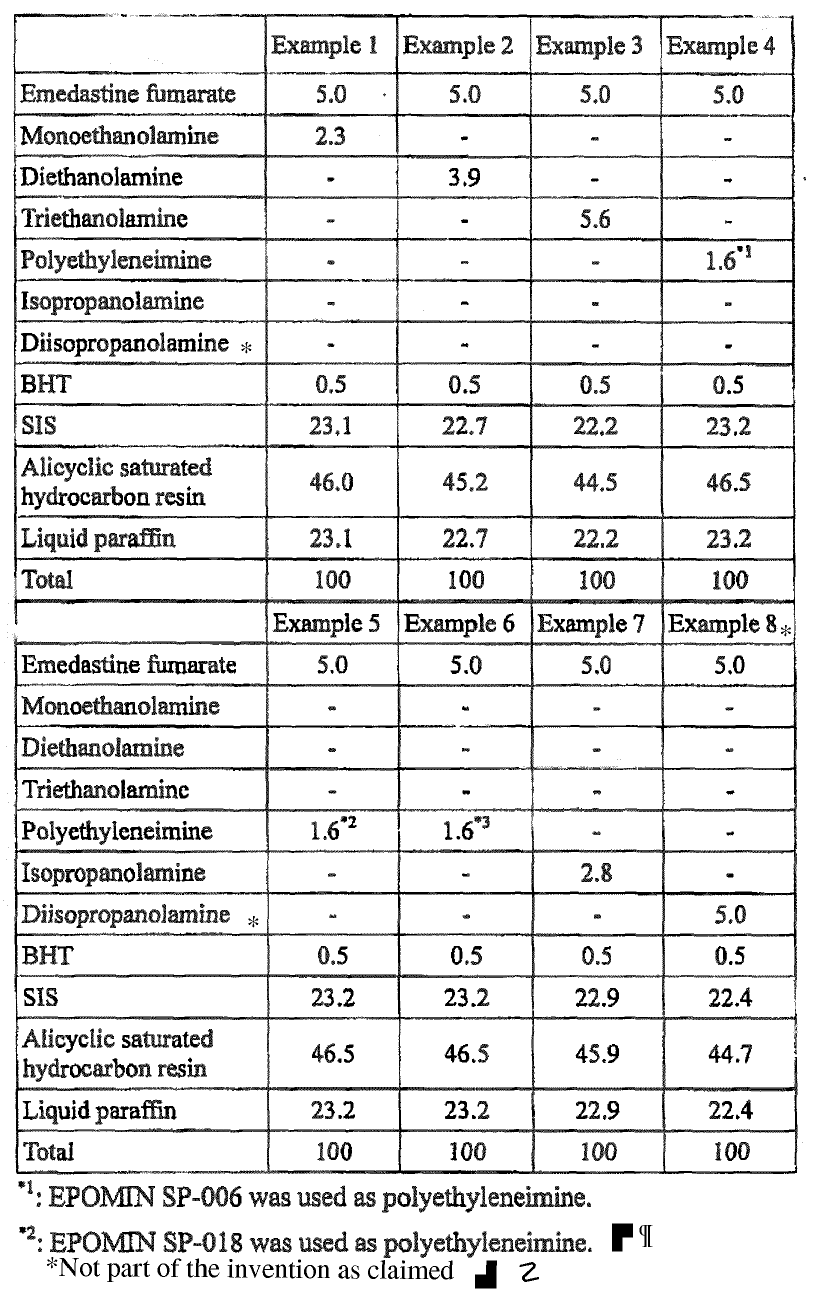

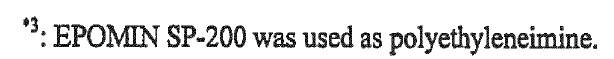

**[Table 2]**

| | Comparative Example 1 | Comparative Example 2 | Comparative Example 3 |
|---|---|---|---|
| Emedastine fumarate | 5.0 | 5.0 | - |
| Emedastine | - | - | 2.8 |
| Sodium hydroxide | 1.5 | - | - |
| BHT | 0.5 | 0.5 | 0.5 |
| SIS | 23.2 | 23.6 | 24.2 |
| Alicyclic saturated hydrocarbon resin | 46.6 | 47.3 | 48.3 |
| Liquid paraffin | 23.2 | 23.6 | 24.2 |
| Total | 100 | 100 | 100 |

A fumarate salt of diethanolamine was produced as follows.

Fumaric acid (0.22 g) and diethanolamine (0.39 g) were added to toluene (0.50 g), and distilled water (0.15 g) was added thereto. The resultant mixture was reacted with stirring overnight. The aqueous phase was collected from the obtained two-phase solution, and the toluene was removed therefrom by drying in a thermostat at 50°C to obtain a fumarate salt of diethanolamine. The obtained fumarate salt of diethanolamine was subjected to measurement of nuclear magnetic resonance spectra (¹H-NMR) to identify the chemical structure. Chemical shifts of the fumarate salt of diethanolamine are shown below. Note that the measurement of ¹H-NMR was conducted using JNM-ECA500 manufactured by JEOL Ltd., and the chemical shifts obtained with tetramethylsilane as the internal standard (0 ppm) were shown. ¹H-NMR (500 MHz, CD₃OD) δ 3.12 (t, 4H), 3.81 (t, 4H), 6.68 (s, 2H). ¹H-NMR (500 MHz, D₂O) δ 3.15 (t, 4H), 3.78 (t, 4H), 6.42 (s, 2H).

Method for producing a tape preparation according to Example 9

Fumaric acid (0.109 g), diethanolamine (0.197 g), and distilled water (0.075 g) were added to toluene and reacted with stirring overnight. Emedastine (0.141 g) and BHT (0.025 g) were then added thereto, and an SIS-PIB base (6.810 g) was added thereto and mixed using a mixer to prepare a homogeneous coating solution. Note that the SIS-PIB base was prepared by mixing the components set forth in Table 4. The values shown in Table 3 denote the masses (unit: g). The obtained coating solution was spread onto a film (releasing film) that had been subjected to the mold release treatment, the solvent was removed by drying to form a pressure-sensitive adhesive layer, a support film was then laminated thereon, and the support film and the pressure-sensitive adhesive layer were pressed and bonded to each other, to thereby obtain a tape preparation. In the obtained tape preparation, therefore, the releasing film, the pressure-sensitive adhesive layer, and the support were laminated in order.

### Method for producing a tape preparation according to Comparative Example 4

Emedastine (0.141 g), BHT (0.025 g), distilled water (0.075 g), and the SIS-PIB base (7.270 g) were added to toluene and mixed using a mixer to prepare a homogeneous coating solution. The obtained coating solution was spread onto a film (releasing film) subjected to the mold release treatment, the solvent was removed by drying to form a pressure-sensitive adhesive layer, a support film was then laminated thereon, and the support film and the pressure-sensitive adhesive layer were compression bonded, thus obtaining a tape preparation. In the obtained tape preparation, therefore, the releasing film, the pressure-sensitive adhesive layer, and the support were laminated in order.

**[Table 3]**

| | Example 9 | Comparative Example 4 |
|---|---|---|
| Emedastine | 0.141 | 0.141 |
| Fumaric acid | 0.109 | - |
| Diethanolamine | 0.197 | - |
| BHT | 0.025 | 0.025 |
| Distilled Water | 0.075 | 0.075 |
| Toluene | 0.050 | 0.050 |
| SIS-PIB Base | 6.810 | 7.270 |

**[Table 4]**

| | Content (mass%) |
|---|---|
| SIS | 15 |
| PIB | 15 |
| Liquid paraffin | 30 |
| Alicyclic saturated hydrocarbon resin | 40 |

### (Dissolution test)

A patch from which a protection film was peeled was placed in the rotary cylinder of a rotary cylinder-type apparatus for dissolution test, so that the pressure-sensitive adhesive layer faced outside. A round bottom flask containing purified water (900 mL) was then placed in the apparatus for dissolution test, and the water temperature was set to 32°C. Next, the rotary cylinder was immersed in the purified water of the round bottom flask, and rotated at a speed of 50 rpm to dissolve emedastine. Then, 10 mL of the dissolution medium was sampled at every predetermined period, and the dissolution ratio (%) was calculated by dividing the amount of emedastine dissolved, which was measured using high performance liquid chromatography, by the emedastine content in the preparation.

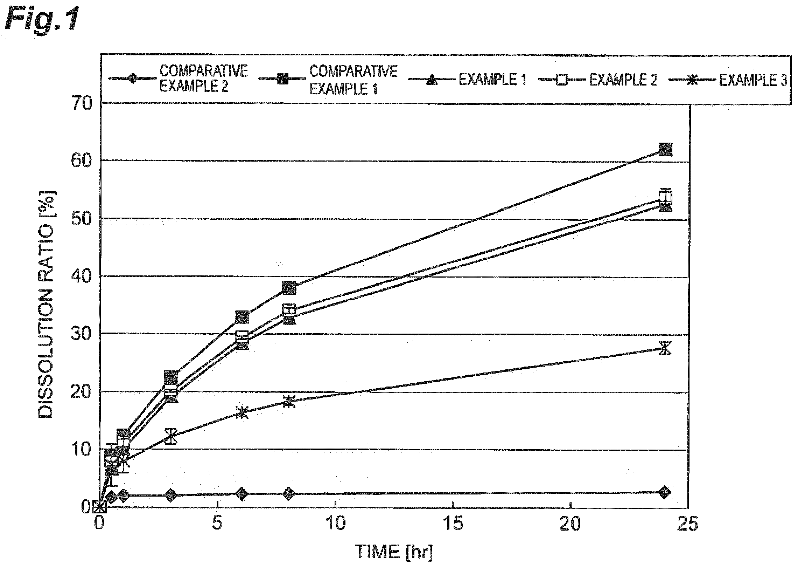

The results are shown in Figure 1. The dissolution ratio of emedastine of the tape preparation according to Comparative Example 2, which does not contain a fumarate salt of an organic amine, was less than 5% even 24 hours after the adhesion, whereas the dissolution ratio of emedastine of the tape preparation according to Comparative Example 1, which contained sodium fumarate, exceeded 60% 24 hours after the adhesion. On the other hand, the dissolution ratio of emedastine of the tape preparation according to Example 1 or 2, which contained the fumarate salt of monoethanolamine or diethanolamine, exceeded 50% 24 hours after the adhesion. The dissolution ratio of emedastine of the tape preparation according to Example 3, which contained the fumarate salt of triethanolamine, was approximately 30% 24 hours after the adhesion.

### (Skin penetrability test)

The skin penetrability of emedastine of each of the tape preparations according to the examples, comparative examples, and reference examples was evaluated as follows.

The dorsal skin of a hairless mouse was removed, and placed in a flow-through cell in which warm water at 32°C was circulated through its outer peripheral portion, so that the dermis side of the removed skin faced the receptor layer side. Next, a patch (adhesion area: 3.0 cm²) was affixed to the stratum corneum-side of the skin, and using, as the receptor layer, physiological saline to which phosphate buffer (pH: 7.4) had been added, the receptor solution was sampled at 1.25 mL/hr every 4 hours until after 24 hours. The flow rate was measured, and simultaneously, the emedastine concentration in the receptor solution was measured using high performance liquid chromatography. The skin penetration rate of the drug (emedastine) per hour was calculated from the measured values, and recorded as the skin penetration rate (µg/cm²/hr) of the drug (emedastine) per unit area in a steady state.

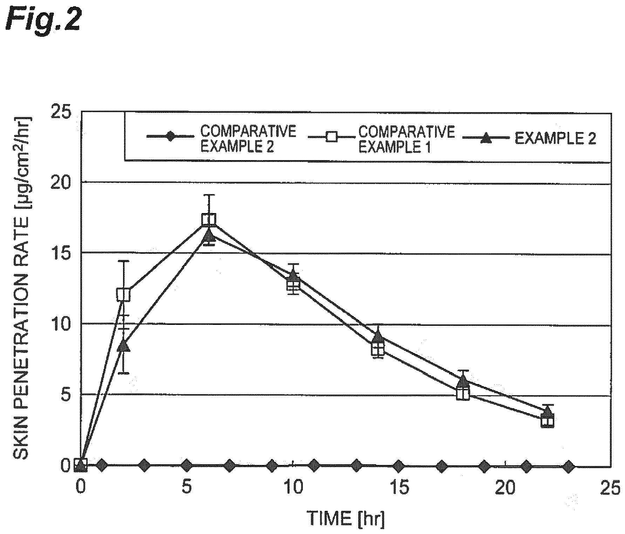

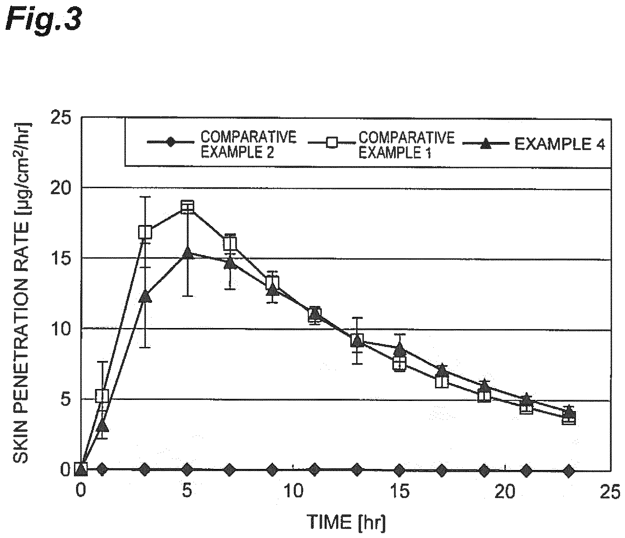

The results are shown in Table 5, and Figures 2 and 3. Note that in Table 5, Jₘₐₓ (maximum skin penetration rate) means the maximum value of the skin penetration rate of the drug (emedastine), within the time during which the measurement was conducted. As shown in Table 5, the tape preparation according to Comparative Example 2, which did not contain a fumarate salt of an organic amine, did not exhibit excellent skin penetrability, and the precipitation of salt was observed in the pressure-sensitive adhesive layer of the tape preparation. Moreover, the tape preparation according to Example 2, which contained the fumarate salt of diethanolamine, and the tape preparation according to Comparative Example 1, which contained sodium fumarate, both exhibited excellent skin penetrability. Furthermore, the tape preparation according to Example 4, which contained the fumarate salt of polyethyleneimine, also achieved excellent skin penetrability, whereas the tape preparation according to Comparative Example 2 did not exhibit excellent skin penetrability.

**[Table 5]**

| | Jmax (µg/cm²/hr) | Cumulative amount of the penetrating drug µg/cm²) | Utilization ratio (%) | State of the preparation |
|---|---|---|---|---|
| Example 2 | 16.3 | 230.0 | 46.0 | Colorless and transparent |
| Example 4 | 15.4 | 236.6 | 47.3 | Yellow and transparent |
| Example 5 | - | - | - | Yellow and transparent |
| Example 6 | - | - | - | Yellow and transparent |
| Comparative Example 1 | 17.3 | 235.4 | 47.1 | Precipitation of salt |
| Comparative Example 2 | 0.0 | 0.2 | 0.0 | Precipitation of salt |
| Example 9 | 11.9 | 221.3 | 44.3 | Colorless and transparent |

### (Peel test)

A 1 cm × 5 cm rectangle sample was cut from each of the tape preparations according to Examples 1 to 8 and Comparative Examples 1 and 3, the releasing film was removed therefrom, and the tape preparation was affixed to a stainless steel plate and allowed to stand for 30 minutes at room temperature. The tape preparation was then peeled at a rate of 300 cm/min, and a five-point average load (unit: gf) was measured. As used herein, the five-point average load is a value obtained by averaging load values measured in a section from the beginning to the end of peeling, from 15 to 85% thereof, at every 17.5% interval. This measurement was repeated three times, and an average value of the obtained five-point average loads was calculated and defined as the peel test value.

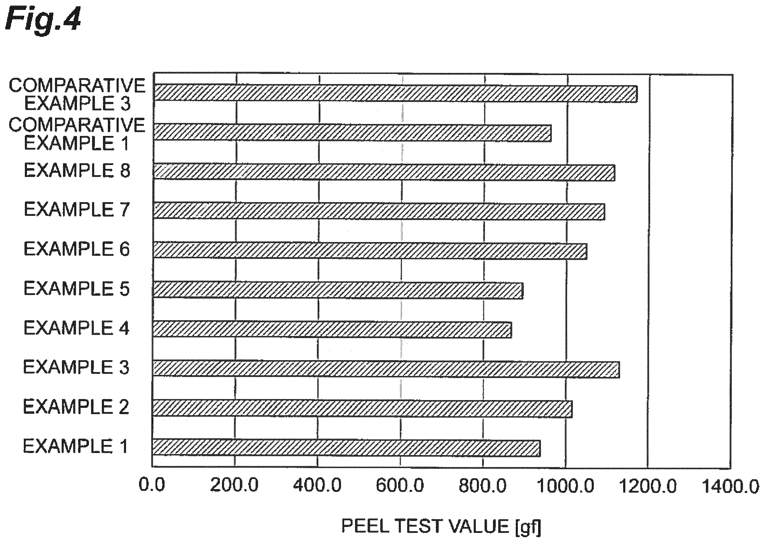

The results are shown in Table 6 and Figure 4. The tape preparations according to Examples 1 to 8 had peel strengths equivalent to those of the tape preparations according to Comparative Examples 1 and 3, and thus had practical peel strengths. In particular, the tape preparations according to Examples 1 to 3 and 6 to 8, Example 8 is not part of the invention as claimed had peel strengths superior to that of the tape preparation according to Comparative Example 1, which contained sodium fumarate.

### (Anchoring property test 1)

For each of the tape preparations according to Examples 1 to 8, Example 8 is not part of the invention as claimed and Comparative Examples 1 and 3, the ratio of the area of the pressure-sensitive adhesive remaining on a stainless steel plate due to anchoring failure (anchoring failure area) when the tape preparation was peeled at 300 cm/min 30 minutes after the adhesion to the stainless steel plate was evaluated by visual observation, and evaluated based on the following evaluation criteria. The anchoring property test was also performed in the similar manner for those in which the material of the support film was polyethylene (PE), polyethylene terephthalate (PET) that had been subjected to a sand matting treatment, or PET that had not been subjected to a sand matting treatment. Note that the anchoring property test was repeated three times, and an average value of the scores was recorded.

### (Evaluation criteria)

0: No anchoring failure.
1: Only an edge of the tape preparation experiences anchoring failure (the anchoring failure area is less than 10% of the adhesion area).
2: The anchoring failure area is not less than 10% and less than 30% of the adhesion area.
3: The anchoring failure area is not less than 30% and less than 50% of the adhesion area.
4: The anchoring failure area is not less than 50% and less than 80% of the adhesion area.
5: The anchoring failure area is not less than 80% and not more than 100% of the adhesion area.

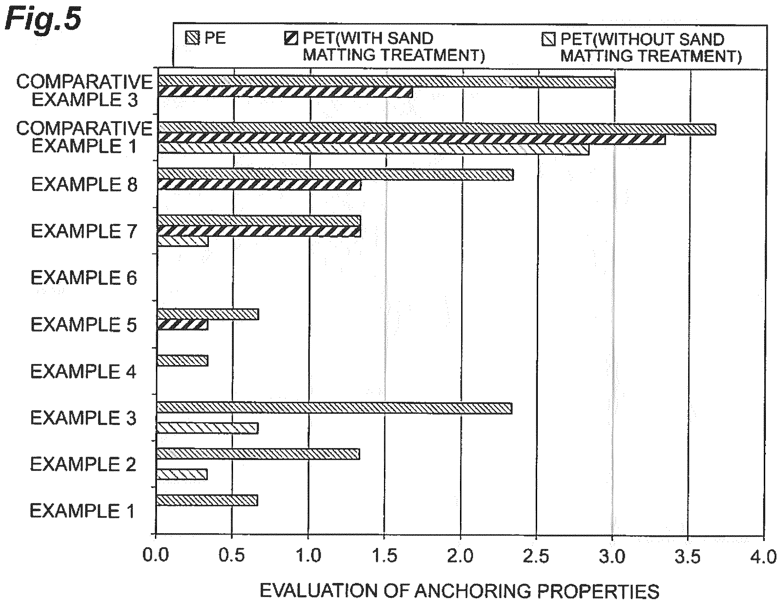

The results are shown in Table 6 and Figure 5. The tape preparations according to Examples 1 to 8, Example 8 is not part of the invention as claimed were superior in anchoring properties to the tape preparations according to Comparative Examples 1 and 3. In particular, when the material of the support film was PE or PET, a significant effect of improving the anchoring properties was obtained.

**[Table 6]**

| | Peel test value [gf] | Anchoring property test | | | |
|---|---|---|---|---|---|
| | | PET (without sand matting treatment) | PET (with sand matting treatment) | PE | EVA |
| Example 1 | 939.0 | 0.0 | 0.0 | 0.7 | 4.7 |
| Example 2 | 1015.0 | 0.3 | 0.0 | 1.3 | 4.3 |
| Example 3 | 1130.4 | 0.7 | 0.0 | 2.3 | 4.3 |
| Example 4 | 866.8 | - | 0.0 | 0.3 | 3.3 |
| Example 5 | 894.0 | - | 0.3 | 0.7 | 4.7 |
| Example 6 | 1048.8 | - | 0.0 | 0.0 | 2.7 |
| Example 7 | 1091.2 | 0.3 | 1.3 | 1.3 | 4.3 |
| Example 8 * | 1115.0 | 0.0 | 1.3 | 2.3 | 5.0 |
| Comparative Example 1 | 960.7 | 2.7 | 2.3 | 3.3 | 4.3 |
| Comparative Example 3 | 1168.2 | 0.0 | 1.7 | 3.0 | 4.7 |

| | | | | | |
|---|---|---|---|---|---|
| * Example 8 is not part of the invention as claimed | | | | | |

The tape preparations according to Examples 1 to 8 * had dissolution ratios and skin penetrability of emedastine, and peel strengths equivalent to those of the tape preparation according to Comparative Example 1, and were superior in anchoring properties to the tape preparation according to Comparative Example 1.

### (Anchoring property test 2)

For each of the tape preparations according to Example 9 and Comparative Example 4, the anchoring failure area of the pressure-sensitive adhesive layer with respect to the support film when the tape preparation was peeled at 300 cm/min 30 minutes after the adhesion to the stainless steel plate was measured and evaluated. The anchoring property test was also performed in the similar manner for those in which the material of the support film was polyethylene (PE), polyethylene terephthalate (PET) that had been subjected to a sand matting treatment, or PET that had not been subjected to a sand matting treatment. Note that the anchoring property test was repeated three times, and an average value of the proportions (unit: %) of the anchoring failure area to the adhesion area was recorded.

**[Table 7]**

| | Peel test value [gf] | Anchoring failure area [%] | | | |
|---|---|---|---|---|---|
| | | PET (without sand matting treatment) | PET (with sand matting treatment) | PE | EVA |
| Example 9 | 1298.3 | 8.6 | 50.4 | 51.4 | 95.6 |
| Comparative Example 4 | 963.9 | 59.7 | 72.3 | 66.3 | 96.3 |

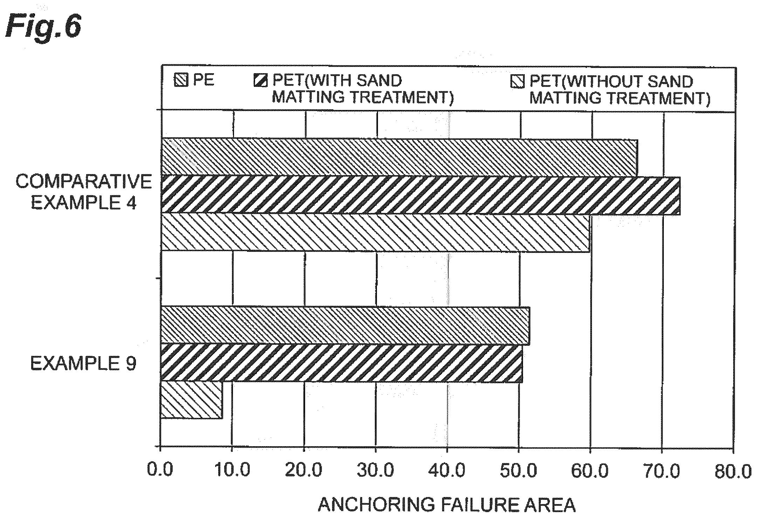

The results are shown in Table 7 and Figure 6. The peel test value of the tape preparation according to Example 9, which contained the fumarate salt of diethanolamine, was equivalent to that of the tape preparation according to Comparative Example 4, which did not contain a fumarate salt of an organic amine. Moreover, the tape preparation according to Example 9, which contained the fumarate salt of diethanolamine, exhibited the effect of suppressing anchoring failure, compared to Example 4.

### Production of tape preparations according to Examples 10 to 12 and Comparative Examples 5 to 7

In accordance with the proportions set forth in Table 8, emedastine fumarate, diethanolamine, sodium hydroxide, BHT, an acrylate pressure-sensitive adhesive having hydroxy groups (DURO-TAK (registered trademark) 87-4287), an acrylate pressure-sensitive adhesive not having a polar functional group (DURO-TAK (registered trademark) 87-901A), and a silicone pressure-sensitive adhesive (PSA7-4302) were mixed using a mixer to prepare a homogeneous coating solution. Note that the values shown in Table 8 each correspond to the percentage by mass (unit: w/w%) of each of the components in the pressure-sensitive adhesive layer. The obtained coating solution was spread onto a film (releasing film) that had been subjected to the mold release treatment, the solvent was removed by drying to form a pressure-sensitive adhesive layer, a support film was then laminated thereon, and the support film and the pressure-sensitive adhesive layer were pressed and bonded to each other, to thereby obtain a tape preparation. In the obtained tape preparation, therefore, the releasing film, the pressure-sensitive adhesive layer, and the support were laminated in order.

**[Table 8]**

| | Example 10 | Comparative Example 5 | Example 11 |
|---|---|---|---|
| Emedastine fumarate | 10.0 | 10.0 | 10.0 |
| Diethanolamine | 7.9 | - | 7.9 |
| Sodium hydroxide | - | 3.0 | - |
| BHT | 0.5 | 0.5 | 0.5 |
| Acrylate pressure-sensitive adhesive having hydroxy groups | 81.6 | 86.5 | - |
| Acrylate pressure-sensitive adhesive not having a polar functional group | - | - | 81.6 |
| Silicone pressure-sensitive adhesive | | | |
| Total | 100 | 100 | 100 |

| | Comparative Example 6 | Example 12 | Comparative Example 7 |
|---|---|---|---|
| Emedastine fumarate | 10.0 | 5.0 | 5.0 |
| Diethanolamine | - | 3.9 | - |
| Sodium hydroxide | 3.0 | - | 1.5 |
| BHT | 0.5 | 0.5 | 0.5 |
| Acrylate pressure-sensitive adhesive having hydroxy groups | - | - | - |
| Acrylate pressure-sensitive adhesive not having a polar functional group | 86.5 | - | - |
| Silicone pressure-sensitive adhesive | - | 90.6 | 93.0 |
| Total | 100 | 100 | 100 |

### (Anchoring property test 3)

Using the tape preparations according to Examples 10 to 12 and Comparative Examples 5 to 7, evaluations were made in the similar manner as in anchoring property test 2.

The results are shown in Table 9. Similarly, in the tape preparations according to Examples 10 and 11, in which the acrylate-based pressure-sensitive adhesive was used as the pressure-sensitive adhesive, the anchoring properties could be improved compared to those of the tape preparations according to Comparative Examples 5 and 6. Moreover, in the case of the tape preparations in which the silicone pressure-sensitive adhesive was used as the pressure-sensitive adhesive, the more remarkable effect of improving the anchoring properties was demonstrated when the material of the support film was EVA. Note that similarly in the case where the material of the support film was PET or PE, the anchoring properties were further enhanced by the inclusion of the fumarate salt of an organic amine.

**[Table 9]**

| | Peel test value [gf] | Anchoring failure area [%] | | | |
|---|---|---|---|---|---|
| | | PET (without sand matting treatment) | PET (with sand matting treatment) | PE | EVA |
| Example 10 | 1488.5 | 25.9 | 8.7 | 20.5 | 97.4 |
| Comparative Example 5 | 1369.8 | 91.3 | 43.7 | 99.2 | 98.4 |
| Example 11 | 435.6 | 21.5 | 21.3 | 49.9 | 98.9 |
| Comparative Example 6 | 1179.7 | 24.1 | 37.7 | 74.3 | 99.1 |
| Example 12 | 757.3 | 0.0 | 0.0 | 0.0 | 0.0 |
| Comparative Example 7 | 685.7 | 2.4 | 0.0 | 5.8 | 62.6 |

## Claims

1. An emedastine-containing tape preparation comprising:
a support film, and a pressure-sensitive adhesive layer laminated on the support film, wherein
the pressure-sensitive adhesive layer comprises emedastine, a fumarate salt of an organic amine, and a pressure-sensitive adhesive.
wherein
the organic amine comprises an organic amine selected from the group consisting of monoethanolamine, diethanolamine, triethanolamine, monoisopropanolamine, and polyethyleneimine.

2. The tape preparation according to claim 1, wherein
the organic amine comprises monoethanolamine or diethanolamine.

3. The tape preparation according to claim 1 or 2, wherein
a material of the support film comprises polyethylene terephthalate or polyethylene.

4. The tape preparation according to any one of claims 1 to 3, wherein
the pressure-sensitive adhesive is a rubber-based pressure-sensitive adhesive.

5. The tape preparation according to claim 4, wherein
the rubber-based pressure-sensitive adhesive comprises a styrene-isoprene-styrene block copolymer.

## Patentansprüche

1. Emedastin-enthaltende Bandzubereitung umfassend:
einen Trägerfilm und eine druckempfindliche Klebemittelschicht, die auf den Trägerfilm laminiert ist, wobei
die druckempfindliche Klebemittelschicht Emedastin, ein Fumaratsalz eines organischen Amins und ein druckempfindliches Klebemittel umfasst,
wobei
das organische Amin ein organisches Amin ausgewählt aus der Gruppe bestehend aus Monoethanolamin, Diethanolamin, Triethanolamin, Monoisopropanolamin und Polyethylenimin umfasst.

2. Bandzubereitung nach Anspruch 1, wobei das organische Amin Monoethanolamin oder Diethanolamin umfasst.

3. Bandzubereitung Anspruch 1 oder 2, wobei ein Material auf dem Trägerfilm Polyethylenterephthalat oder Polyethylen umfasst.

4. Bandzubereitung nach einem der Ansprüche 1 bis 3, wobei das druckempfindliche Klebemittel ein Kautschuk-basiertes druckempfindliches Klebemittel ist.

5. Bandzubereitung nach Anspruch 4, wobei das Kautschuk-basierte druckempfindliches Klebemittel ein Styrol-Isopren-Styrol-Blockcopolymer umfasst.

## Revendications

1. Préparation pour bande contenant de l'émédastine comprenant :
un film de support, et une couche adhésive sensible à la pression stratifiée sur le film de support, où
la couche adhésive sensible à la pression comprend de l'émédastine, un sel fumarate d'une amine organique, et un adhésif sensible à la pression, où
l'amine organique comprend une amine organique sélectionnée dans le groupe constitué de la monoéthanolamine, diéthanolamine, triéthanolamine, monoisopropanolamine, et de la polyéthylène imine.

2. Préparation pour bande selon la revendication 1, dans laquelle
l'amine organique comprend de la monoéthanolamine ou de la diéthanolamine.

3. Préparation pour bande selon la revendication 1 ou 2, dans laquelle
un matériau du film de support comprend du poly(téréphtalate d'éthylène) ou du polyéthylène.

4. Préparation pour bande selon l'une quelconque des revendications 1 à 3, dans laquelle
l'adhésif sensible à la pression est un adhésif sensible à la pression à base de caoutchouc.

5. Préparation pour bande selon la revendication 4, dans laquelle
l'adhésif sensible à la pression à base de caoutchouc comprend un copolymère séquencé de styrène-isoprène-styrène.
